(19) Europäisches Patentamt
European Patent Office
Office européen des brevets

(11) Veröffentlichungsnummer: **0 021 000**
**B1**

(12) # EUROPÄISCHE PATENTSCHRIFT

(45) Veröffentlichungstag der Patentschrift: **22.02.84**

(21) Anmeldenummer: **80102661.8**

(22) Anmeldetag: **13.05.80**

(51) Int. Cl.³: **C 07 C 49/84,** C 07 C 69/035, C 07 C 69/157, C 07 C 69/78, C 07 C 69/96, C 07 C 101/19, C 07 D 235/08, C 07 D 307/68, C 07 D 317/54, C 07 F 9/09, C 07 H 15/20

(54) **Substituierte Acetophenone, deren Herstellung und pharmazeutische Präparate, die sie enthalten.**

(30) Priorität: **23.05.79 GB 7917973**

(43) Veröffentlichungstag der Anmeldung: **07.01.81 Patentblatt 81/1**

(45) Bekanntmachung des Hinweises auf die Patenterteilung: **22.02.84 Patentblatt 84/8**

(84) Benannte Vertragsstaaten: **AT BE CH DE FR GB IT LI LU NL SE**

(56) Entgegenhaltungen:
**DD - A - 123 466**
**DE - A - 2 010 180**
**DE - A - 2 240 226**
**DE - B - 1 291 746**
**DE - C - 964 680**
**FR - A - 1 476 531**
**FR - A - 2 031 452**
**US - A - 3 583 894**
**US - A - 4 089 606**

**Chemical Abstracts Band 81, Nr. 23, 9. Dezember 1974, Columbus, Ohio, USA; A.W. FRASER et al. "Rutaceae constituents. IV.Flavonoids from Merrillia caloxylon", Seite 247, Spalte 2, Abstract Nr. 148530r.**

(73) Patentinhaber: **F. HOFFMANN-LA ROCHE & CO. Aktiengesellschaft**
**CH-4002 Basel (CH)**

(72) Erfinder: **Umeda, Isao**
**204 Height Hakuraku 4-4-20 Rokkakubashi**
**Yokohama-shi, Kanagawa-ken (JP)**
Erfinder: **Ishitsuka, Hideo**
**1-1-go, 405, Katsura-cho 1-banchi**
**Totsuka-ku Yokohama-shi, Kanagawa-ken (JP)**
Erfinder: **Shinma, Nobuo**
**16 Renaissance Drive**
**Clifton, N.J. (US)**
Erfinder: **Suhara, Yasuji**
**Dream Height 9-506 Matano-cho, 1403-banchi**
**Totsuka-ku Yokohama-shi, Kanagawa-ken (JP)**

(74) Vertreter: **Lederer, Franz, Dr. et al,**
**Patentanwählte Dr. Franz Lederer Reiner F. Meyer**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 021 000 B1

Substituierte Acetophenone, deren Herstellung und pharmazeutische Präparate, die sie enthalten.

Die vorliegende Erfindung betrifft neue substituierte Acetophenone, ein Verfahren zu deren Herstellung und antivirale Mittel auf der Basis dieser Verbindungen.

Die vorliegende Erfindung betrifft insbesondere neue substituierte Acetophenone der allgemeinen Formel

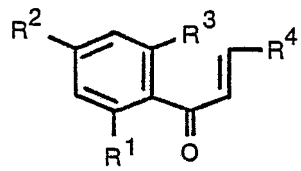

I

worin

$R^1$ Hydroxy, Phosphonooxy, Benzyloxycarbonyloxy, Trialkylammonioacyloxy, Furoyloxy, ggf. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-, Niederalkoxy-, Alkylthio-, Alkylendioxy-, Acyloxy- oder Alkoxyalkoxy-substituiertes Phenyl darstellen. mit der Einschränkung, dass, wenn $R^1$ Hydroxy ist,

$R^4$ Benzimidazolyl oder Acyloxy-substituiertes Phenyl bedeutet.

Die Verbindungen der Formel I können als antivirale Mittel Verwendung finden.

Bevorzugte Beispiele von ggf. acylierten Aminoglycosyloxygruppen sind Peracetyl-$\beta$-D-glucosaminyloxy und N-Acetyl-$\beta$-D-glucosaminyloxy. Bevorzugte, ggf. substituierte Arylcarbonyloxygruppen sind Benzoyloxy, 4-Methoxybenzoyloxy und 2,4-Dimethoxybenzoyloxy. Trialkylammonioacyloxy ist vorzugsweise Triethylammonioacetoxy. Niederalkoxygruppen enthalten vorzugsweise 1—4 C-Atome, wie Methoxy, Ethoxy, Propoxy und Butoxy. Bevorzugte Beispiele ggf. Niederalkyl-substituierter Furyl- oder Thienylgruppen sind 2-Furyl, 5-Methyl-2-furyl oder 2-Thienyl. Bevorzugte substituierte Phenylreste sind p-Tolyl, p-Methoxyphenyl, p-Ethoxyphenyl, p-Methoxymethoxyphenyl, p-Acetoxyphenyl, m,p-(Methylendioxy)-phenyl und p-(Methylthio)-phenyl.

Repräsentative Verbindungen der Formel I sind:

2′,4,4′-Trimethoxy-6′-(phosphonooxy)-chalkon;
4-Ethoxy-2′,4′-dimethoxy-6′-(phosphonooxy)-chalkon;
2′,4′-Dimethoxy-4-methyl-6′-(phosphonooxy)-chalkon;
2′,4′-Dimethoxy-4-(methylthio)-6′-(phosphonooxy)chalkon;
2′-Ethoxy-4,4′-dimethoxy-6′-(phosphonooxy)-chalkon;
2′,4′-Dimethoxy-3-(5-methyl-2-furyl)-6′-(phosphonooxy)-acrylophenon;
4′-Ethoxy-2′,4-dimethoxy-6′-(phosphonooxy)-chalkon;
2′,4′-Dimethoxy-3,4-(methylendioxy)-6′-(phosphonooxy)-chalkon;
2′,4′-Dimethoxy-6′-(phosphonooxy)-3-(2-thienyl)acrylophenon;
2′-(2-Acetamido-2-deoxy-3,4,6-tri-O-acetyl-$\beta$-D-glucopyranosyloxy)-4,4′,6′-trimethoxychalkon;
2′-(2-Acetamido-2-deoxy-$\beta$-D-glucopyranosyloxy)-4,4′,6′-trimethoxychalkon;
2′-(Benzyloxycarbonyloxy)-4,4′,6′-trimethoxychalkon;
2′,4,4′-Trimethoxy-6′-(triethylammonioacetoxy)-chalkon;
2′,4,4′-Trimethoxy-6′-(4-methoxybenzoyloxy)-chalkon;
2′-(2,4-Dimethoxybenzoyloxy)-4,4′,6′-trimethoxychalkon;
2′-(Benzoyloxy)-4,4′,6′-trimethoxychalkon;
2′-(2-Furoyloxy)-4,4′,6′-trimethoxychalkon;
3-(5-Benzimidazolyl)-2′-hydroxy-4′,6′-dimethoxyacrylophenon;
4-Acetoxy-2′-hydroxy-4′,6′-dimethoxychalkon.

Erfindungsgemäss können die Verbindungen der Formel I dadurch hergestellt werden, dass man

(a) eine Verbindung der Formel

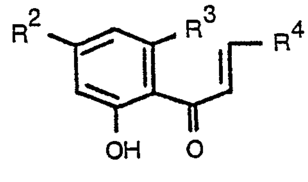

II

worin $R^2$, $R^3$ und $R^4$ die oben angegebenen Bedeutungen besitzen, mit einem Phosphoroxyhalogenid in einem Lösungsmittel in Gegenwart einer Base umsetzt und das Reaktionsprodukt hydrolysiert oder

(b) eine Verbindung der Formel II mit einem 2-Acetamido-2-deoxy-3,4,6-tri-O-acetyl-$\alpha$-D-

glucopyranosyl-halogenid in einem Lösungsmittel in Gegenwart eines Alkalimetallhalogenids umsetzt und gewünschtenfalls das Reaktionsprodukt partiell hydrolysiert oder

(c) eine Verbindung der Formel II mit einem Trialkylamin und Haloacetylhalogenid in einem Lösungsmittel umsetzt oder

(d) die Hydroxygruppe einer Verbindung der Formel II mit einem reaktiven Derivat einer Benzyloxycarbonsäure, einer ggf. substituierten Arylcarbonsäure oder einer Furancarbonsäure acyliert oder

(e) die Hydroxygruppe in 4-Stellung einer Verbindung der Formel

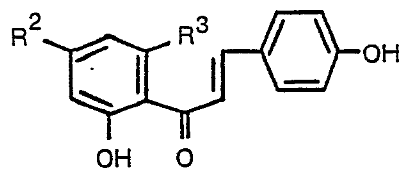

III

worin $R^2$ und $R^3$ die oben angegebenen Bedeutungen besitzen, mit einem reaktiven Derivat einer Alkancarbonsäure acyliert oder

(f) eine Verbindung der Formel

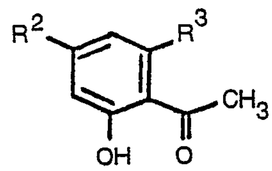

IV

worin $R^2$ und $R^3$ die oben angegebenen Bedeutungen besitzen, mit einem Aldehyd der Formel

$$R^4\text{—CHO} \qquad V$$

worin $R^4$ die oben angegebene Bedeutung besitzt, in einem Lösungsmittel in Gegenwart eines basischen Katalysators umsetzt.

Die Durchführung der Verfahrensvariante (a) kann dadurch erfolgen, dass man eine Verbindung der Formel II mit einem Phosphoroxyhalogenid, wie Phosphoroxychlorid oder Phosphoroxybromid, in einem Lösungsmittel, wie Benzol Toluol, Tetrahydrofuran, Dioxan oder Cyclohexan, in Gegenwart einer Base, wie N,N-Diisopropylethylamin, Triethylamin oder Pyridin, umsetzt und das erhaltene Produkt in an sich bekannter Weise hydrolysiert. Die Verbindungen der Formel II können gemäss Verfahrensvariante (f) der vorliegenden Erfindung hergestellt werden.

Die Umsetzung gemäss Verfahrensvariante (b) kann dadurch erfolgen, dass man 2-Acetamido-2-deoxy-3,4,6-tri-O-acetyl-α-D-glucopyranosyl-halogenid zu einer Lösung einer Verbindung der Formal II und einem Alkalihydrid, wie Natriumhydrid oder Kaliumhydrid, in einem Lösungsmittel, wie Dimethylformamid, Dioxan, Tetrahydrofuran oder Benzol, umsetzt. Hydrolysiert man das erhaltene Produkt in an sich bekannter Weise, so werden die Acetylreste ausser dem Acetamidorest entfernt.

Die Umsetzung gemäss Verfahrensvariante (c) kann dadurch erfolgen, dass man ein Trialkylamin und ein Haloacetylhalogenid, beispielsweise Triethylamin und Bromacetylbromid, zu einer Lösung einer Verbindung der Formel II in einem organischen Lösungsmittel, z.B. Ethylacetat, zugibt.

Die Acylierung der Hydroxylgruppe einer Verbindung der Formel II gemäss Verfahrensvariante (d) kann dadurch erfolgen, dass man in an sich bekannter Weise eine Verbindung der Formel II mit einem reaktiven Derivat einer Benzyloxycarbonsäure, einer substituierten oder unsubstituierten Arylcarbonsäure oder einer Furancarbonsäure umsetzt. Bevorzugte reaktive Derivate sind Bis-(4-methoxybenzoesäure)anhydrid, Benzoylchlorid, Benzyloxycarbonylchlorid, 2,4-Dimethoxybenzoylchlorid oder 2-Furoylchlorid.

Die Acylierung der Hydroxylgruppe in 4-Stellung einer Verbindung der Formel III gemäss Verfahrensvariante (e) kann ebenso in an sich bekannter Weise durch Behandlung mit einem reaktiven Derivat einer Alkancarbonsäure, beispielsweise Acetanhydrid, erfolgen.

Die Umsetzung gemäss Verfahrensvariante (f) kann dadurch erfolgen, dass man einen basischen Katalysator, beispielswiese ein Alkalimetallhydroxid oder -carbonat wie Natriumhydroxid, Kaliumhydroxid, Natriumcarbonat oder Kaliumcarbonat oder ein Alkoholat wie Natriumethoxid oder Kaliumethoxid, zu einer Lösung der Verbindungen IV und V in einem organischen Lösungsmittel, wie Methanol, Ethanol, Dioxan, Tetrahydrofuran, Benzol oder Hexan, gibt und das Gemisch mehrere Stunden bis zu 3 Tagen bei einer Temperatur von 0 bis etwa 100°C rührt.

Die vorliegende Erfindung betrifft auch antivirale Mittel auf der Basis eines substituierten Acetophenonderivats der Formel I. Die Verbindungen der Formel I sind insbesondere wirksam gegen bestimmte Viren der Gruppe Picarna. Besonders geeignet für nasale Administration bzw. zur Herstellung von injektionen sind auf Grund ihrer Wasserlöslichkeit die folgenden Verbindungen:

2',4,4'-Trimethoxy-6'-(phosphonooxy)-chalkon;

4-Ethoxy-2',4'-dimethoxy-6'-(phosphonooxy)-chalkon;

2′,4′-Dimethoxy-4-methyl-6′-(phosphonooxy)-chalkon;
2′,4′-Dimethoxy-4-(methylthio)-6′-(phosphonooxy)-chalkon;
2′-Ethoxy-4,4′-dimethoxy-6′-(phosphonooxy)-chalkon;
2′,4′-Dimethoxy-3-(5-methyl-2-furyl)-6′-(phosphonooxy)-acrylophenon;
4′-Ethoxy-2′,4-dimethoxy-6′-(phosphonooxy)-chalkon;
2′,4′-Dimethoxy-3,4-(methylendioxy)-6′-(phosphonooxy)-chalkon;
2′,4′-Dimethoxy-6′-(phosphonooxy)-3-(2-thienyl)-acrylphenon;
2′-(2-Acetamido-2-deoxy-β-D-glucopyranosyloxy)-4,4′,6′-trimethoxychalkon;
2′,4,4′-Trimethoxy-6′-(triethylammoniumacetoxy)-chalkon.

Die Verbindungen der Formel I besitzen antivirale Aktivität und hemmen insbesondere die Replikation von menschlichen Rhinoviren in Zellkulturen menschlicher embryonaler Lungenzellen oder HeLa-Zellen bei Konzentrationen von 0,006 bis etwa 1 μg/ml.

Im folgenden sind die Testergebnisse von Untersuchungen der antiviralen Aktivität zusammengefasst:

1. In vitro antivirale Aktivität (Hemmung des viralen cytopathogenen Effekts)

Eine Suspension von HeLa-Zellen (6 x $10^4$) wurde mit Rhinoviren HGP (3 x $10^3$ koloniebildende Einheiten (PFU)) vermischt und auf eine Mikrotestplatte aufgetragen, die die zu testenden Verbindungen in Verdünnungsreihen enthielt. Die Zellen wurden anschliessend mit Eagel's essentiellem Minimalmedium, enthaltend 2% Kälberserum, 1% Tryptosephosphatbrühe, 100 μg/ml Streptomycin und 20 Einheiten/ml Penicillin G, kultiviert. Der virale cytopathogene Effekt wurde unter dem Mikroskop beobachtet und zwar nach 2 Tagen Kultur bei 33°C.

Die erhaltenen Resultate sind in der Tabelle 1 zusammengefasst. Die antivirale Aktivität der getesteten Verbindungen wird angegeben als Konzentration, die nötig ist, um den viralen cytopathogenen Effekt, verglichen mit einer Kontrollkultur, um 50% zu hemmen ($IC_{50}$).

TABELLE 1

| Verbindung | $IC_{50}$ (µg/ml) Rhinovirus HGP |
|---|---|
| 2',4,4'-Trimethoxy-6'-(phosphonooxy)-chalkon | 0.03—0.1 |
| 4-Ethoxy-2'-4'-dimethoxy-6'-(phosphonooxy)-chalkon | 0.006—0.02 |
| 2',4'-Dimethoxy-4-methyl-6'-(phosphonooxy)-chalkon | 0.02 |
| 2',4'-Dimethoxy-4-(methylthio)-6'-(phosphonooxy)-chalkon | 0.02 |
| 2'-Ethoxy-4,4'-dimethoxy-6'-(phosphonooxy)-chalkon | 0.02 |
| 2',4'-Dimethoxy-3-(5-methyl-2-furyl)-6'-(phosphonooxy)-acrylophenon | 0.04—0.1 |
| 4'-Ethoxy-2',4-dimethoxy-6'-(phosphonooxy)-chalkon | 0.03 |
| 2',4'-Dimethoxy-3,4-(methylendioxy)-6'-(phosphonooxy)-chalkon | 0.06 |
| 2',4'-Dimethoxy-6'-(phosphonooxy)-3-(2-thienyl)-acrylophenon | 0.04—0.1 |
| 2'-(2-Acetamido-2-deoxy-3,4,6-tri-O-acetyl-β-D-glucopyranosyloxy)-4,4',6'-trimethoxychalkon | 0.01—0.3 |
| 2'-(2-Acetamido-2-deoxy-β-D-glucopyranosyloxy)-4,4',6'-trimethoxychalkon | 3—10 |
| 2'-(Benzyloxycarbonyloxy)-4,4',6'-trimethoxychalkon | 0.01—0.03 |
| 2',4,4'-Trimethoxy-6'-(triethylammonioacetoxy)-chalkonchlorid | 0.01 |
| 2',4,4'-Trimethoxy-6'-(4-methoxybenzoyloxy)-chalkon | 0.03—0.1 |
| 2'-(2,4-Dimethoxybenzoyloxy)-4,4',6'-trimethoxychalkon | 0.03—0.1 |
| 2'-(benzoyloxy)-4,4',6'-trimethoxychalkon | 0.03—0.1 |
| 2'-(2-furoyloxy)-4,4',6'-trimethoxychalkon | 0.03 |
| 3-(5-Benzimidazolyl)-2'-hydroxy-4',6'-dimethoxyacrylophenon | 0.01—0.3 |
| 4-Acetoxy-2'-hydroxy-4',6'-dimethoxychalkon | 0.3 |

2. In vivo antivirale Aktivität

Die antivirale Aktivität der in der folgenden Tabelle II aufgeführten Verbindungen wurde gegen lethale Infektionen mit Coxsackie-Virus B1 an Mäusen getestet. 15 g schwere ddy Mäuse wurden intraperitoneal mit etwa der zehnfachen $LD_{50}$ der Viren infiziert. Die infizierten Mäuse wurden anschliessend viermal, und zwar 2 Stunden vor und 7, 22 und 31 Stunden nach der Infektion, mit den Verbindungen entweder oral oder intravenös behandelt. Die überlebenden Tiere wurden bis zum 21. Tag gezählt. Die nicht behandelten Mäuse starben zwischen dem 3. und 5. Tag nach der Infektion.

TABELLE 2

Antivirale Aktivität gegen Coxsackie-Virus B1 an Mäusen

| Verbindung | Dosis | überlebende Tiere [%] |
|---|---|---|
| 2′,4,4′-Trimethoxy-6′-(phosphonooxy)-chalkon | 80 mg/kg × p.o. | 70 |
| | 40 | 50 |
| | 20 | 10 |
| 2′,4,4′-Trimethoxy-6′-(phosphonooxy)-chalkon | 10 mg/kg × 4 i.v. | 60 |
| | 5 | 20 |
| 2′-Ethoxy-4,4′-dimethoxy-6′-(phosphonooxy)-chalkon | 40 mg/kg × 4 p.o. | 30 |

Darüberhinaus werden die erfindungsgemässen Verbindungen gut vertragen und zeigen keinerlei toxische Effekte bei Konzentrationen, die 10 bis 1000 × höher sind als die Konzentrationen, die eine antivirale Aktivität bei Rhinovirus-Infektionen bewirken. Bei oraler Verabreichung an Mäusen bewirken die Verbindungen keine toxischen Symptome bei Dosen von 1 g/kg Körpergewicht oder darüber. Die folgende Tabelle 3 enthält Angaben über die akute Toxizität bei Mäusen.

TABELLE 3

| Verbindung | $LD_{50}$ (mg/kg)[1] | |
|---|---|---|
| | i.p.[2] | p.o.[3] |
| 2',4,4'-Trimethoxy-6'-(phosphonooxy)-chalkon | >500 | >1.000 |
| 4-Ethoxy-2',4'-dimethoxy-6'-(phosphonooxy)-chalkon | >500 | >1.000 |
| 2',4'-Dimethoxy-4-methyl-6'-(phosphonooxy)-chalkon | >500 | >2.000 |
| 2',4'-Dimethoxy-4-(methylthio)-6'-(phosphonooxy)-chalkon | >500 | >1.000 |
| 2'-Ethoxy-4,4'-dimethoxy-6'-(phosphonooxy)-chalkon | >500 | >1.000 |
| 2',4'-Dimethoxy-3-(5-methyl-2-furyl)-6'-(phosphonooxy)-acrylophenon | >500 | >1.200 |
| 4'-Ethoxy-2',4-dimethoxy-6'-(phosphonooxy)-chalkon | >500 | >2.000 |
| 2',4'-Dimethoxy-3,4-(methylendioxy)-6'-(phosphonooxy)-chalkon | >500 | >1.000 |
| 2',4'-Dimethoxy-6'-(phosphonooxy)-(2-thienyl)-acrylophenon | >500 | >1.000 |
| 2'-(2-Acetamido-2-deoxy-3,4,6-tri-O-acetyl-β-D-glucopyranosyloxy)-4,4',6'-trimethoxychalkon | >500 | >2.000 |
| 2'-(2-Acetamido-2-deoxy-β-D-glucopyranosyloxy)-4,4',6'-trimethoxychalkon | >300 | >2.000 |
| 2'-(Benzyloxycarbonyloxy)-4,4',6'-trimethoxychalkon | >500 | >1.000 |
| 2',4,4'-Trimethoxy-6'-(triethylammonioacetoxy)-chalkonchlorid | >180 | >1.000 |
| 2',4,4'-Trimethoxy-6'-(4-methoxybenzoyloxy)-chalkon | >500 | >1.000 |
| 2'-(2,4-Dimethoxybenzoyloxy)-4,4',6'-trimethoxy-chalkon | >500 | >1.000 |
| 2'-(Benzoyloxy)-4,4',6'-trimethoxychalkon | >500 | >1.000 |
| 2'-(2-Furoyloxy)-4,4',6'-trimethoxychalkon | >500 | >1.000 |
| 3-(5-Benzimidazolyl)-2'-hydroxy-4',6'-dimethoxyacrylophenon | >100 | >1.000 |
| 4-Acetoxy-2'-hydroxy-4',6'-dimethoxychalkon | >500 | >1.000 |

1) Den 15—20 g schweren ddy-Mäusen wurde eine einmalige Dosis der entsprechenden Verbindung verabreicht. Die überlebenden Tiere wurden nach 21 Tagen gezählt.
2) Die Verbindungen wurden in Dimethylsulfoxid gelöst.
3) Die Verbindungen wurden in einer Lösung von 0,5% Carboxymethylcellulose suspendiert.

Wie bereits oben erwähnt, können die Verbindungen der Formel I als Medikamente gegen virale

Erkrankungen, insbesondere gegen Erkältungen, in Form pharmazeutischer Präparate verwendet werden.

Die pharmazeutischen Präparate enthalten mindestens eine der erwähnten antiviral wirksamen Verbindungen zusammen mit einem verträglichen pharmazeutischen Träger. Dieser Träger kann ein für die enterale, perkutane oder parenterale Verabreichung geeignetes organisches oder anorganisches Trägermaterial sein, wie z.B. Wasser, Gelatine, Gummi arabicum, Lactose, Stärke, Magnesiumstearat, Talk, pflanzliche Oele, Polyalkylenglycole, Vaseline und dergleichen. Darüberhinaus können die pharmazeutischen Präparate weitere pharmazeutisch wertvolle Stoffe enthalten, wie fiebersenkende Mittel, schmerszstillende Mittel, entzündungshemmende Mittel, Antihistamine, Interferonstarter und dergleichen. Die pharmazeutischen Präparate können oral, z.B. in Form von Tabletten, Kapseln, Pillen, Pulvern, Granulaten, Lösungen, Sirupen, Suspensionen, Elixieren und dergleichen verabreicht werden. Die Verabreichung kann aber auch parenteral, z.B. in Form von sterilen Lösungen, Suspensionen oder Emulsionen, oder lokal, in Form von Lösungen, Suspensionen, Salben, Pudern, Aerosolen, und dergleichen, erfolgen.

Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die pharmazeutischen Präparate können so verabreicht werden, dass die Konzentration des aktiven Wirkstoffes grösser ist als die erforderliche minimale Hemmkonzentration gegenüber den zu bekämpfenden Viren.

Die Behandlungsdosis ist abhängig von der Verabreichungsart, vom Alter, Gewicht und Befinden des Patienten und insbesondere von der Krankheit, die behandelt werden soll. Im allgemeinen liegt für Erwachsene die Dosis bei Behandlungen von Erkältungskrankheiten bei 100 bis 2000 mg, oral, 3 bis 6 mal täglich, und bei etwa 0,1 bis 100 $\mu g/cm^2$, topisch, 3 bis 6 mal täglich.

Die folgenden Beispiele erläutern die vorliegende Erfindung, ohne sie jedoch einzuschränken.

## Beispiel 1

Zu einer Lösung von 1,0 g 2'-Hydroxy-4,4',6'-trimethoxychalkon und 2 ml N,N-Diisopropylethylamin in 20 ml wasserfreiem Toluol wurden unter Rühren 10 ml Phosphoroxychlorid gegeben. Das Gemisch wurde 50 Minuten bei Raumtemperatur gerührt, unter vemindertem Druck bei einer Badtemperatur von 30—40°C eingeengt und lieferte eine braune ölige Substanz, die über Nacht über Phosphorpentoxid getrocknet wurde. Dann wurde durch Behandlung mit 50 ml eines Gemisches aus Wasser und Tetrahydrofuran (1:1 v/v) während 10 Minuten bei Raumtemperatur hydrolysiert. Das organische Lösungsmittel wurde unter vermindertem Druck bei einer Badtemperatur von 30—40°C entfernt, das wässrige Konzentrat dreimal mit je 50 ml eiskaltem Chloroform extrahiert, die vereinigten Extrakte über Natriumsulfat getrocknet und unter vermindertem Druck zu einem bräunlichen Rückstand eingeengt. Der Rückstand wurde gelöst in 30 ml einer 0,1N Kaliumcarbonatlösung diese wurde zweimal mit je 50 ml Aethylacetat gewaschen, angesäuert mit kalter Salzsäure und dreimal mit je 50 ml Chloroform extrahiert. Die vereinigten Extrakte wurden über Natriumsulfat getrocknet und unter vermindertem Druck auf ein Volumen von etwa 10 ml gebracht. Das Konzentrat wurde auf eine Kieselsäule gebracht (40 g) die mit Chloroform-Methanol (10:1, v/v) eluiert wurde. Die Fraktionen 30—50 (zu je 10 ml) wurden vereinigt und unter vermindertem Druck zu 0,65 g eines gelben Rückstandes eingeengt. Kristallisation des Rückstandes aus Ethanol-Hexan lieferte 0,55 g (Ausbeute 44%) 2',4,4'-Trimethoxy-6'-(phosphonooxy)-chalkon in Form rötlichbrauner Kristalle; Fp. 71—73°C.

## Beispiel 2

In zu Beispiel 1 analoger Weise wurde aus 4-Ethoxy-2'-hydroxy-4',6'-dimethoxychalkon 4-Ethoxy-2',4'-dimethoxy-6'-(phosphonooxy)-chalkon, Fp. 63—64°C, erhalten.

## Beispiel 3

In zu Beispiel 1 analoger Weise wurde aus 2'-Hydroxy-4',6'-dimethoxy-4-methylchalkon, 2',4'-Dimethoxy-4-methyl-6'-(phosphonooxy)-chalkon, Fp. 81—83°C, erhalten.

## Beispiel 4

In zu Beispiel 1 analoger Weise wurde aus 2'-Hydroxy-4',6'-dimethoxy-4-(methylthio)-chalkon, 2',4'-Dimethoxy-4-(methylthio)-6'-(phosphonooxy)-chalkon, Fp. 63—66°C, erhalten.

## Beispiel 5

In zu Beispiel 1 analoger Weise wurde aus 2'-Ethoxy-6'-hydroxy-4,4'-dimethoxychalkon 2'-Ethoxy-4,4'-dimethoxy-6'-(phosphonooxy)-chalkon, Fp. 72—75°C, erhalten.

## Beispiel 6

In zu Beispiel 1 analoger Weise wurde aus 2'-Hydroxy-4',6'-dimethoxy-3-(5-methyl-2-furyl)-acrylophenon 2',4'-Dimethoxy-3-(5-methyl-2-furyl)-6'-(phosphonooxy)-acrylophenon erhalten: $^1H$—NMR($CDCl_3$) $\delta$3,8(3H, $OCH_3$), 3,85 (3H, $OCH_3$) und 2,9 ppm (3H, $CH_3$).

Beispiel 7

In zu Beispiel 1 analoger Weise wurde aus 4′-Ethoxy-2′-hydroxy-4,6′-dimethoxychalkon 4′-Ethoxy-2′-4-dimethoxy-6′-(phosphonooxy)-chalkon, Fp. 85—88°C, erhalten.

Beispiel 8

In zu Beispiel 1 analoger Weise wurde aus 2′-Hydroxy-4′,6′-dimethoxy-3,4-(methylendioxy)-chalkon 2′,4′-Dimethoxy-3,4-(methylendioxy)-6′-(phosphonooxy)-chalkon Fp. 79—80°C, erhalten.

Beispiel 9

In zu Beispiel 1 analoger Weise wurde aus 2′-Hydroxy-4′,6′-dimethoxy-3-(2-thienyl)-acrylphenon 2′,4′-Dimethoxy-6′-(phosphonooxy)-3-(2-thienyl)-acrylophenon erhalten: $^1H$—NMR ($CDCl_3$) δ3,5 (3H, $OCH_3$), 3,6 (3H, $OCH_3$) und 7,0 ppm (1H, Thienyl-Proton in 3-Stellung).

Beispiel 10

Zu einem Gemisch aus 500 mg 2′-Hydroxy-4,4′,6′-trimethoxychalkon und 100 mg Natriumhydrid (Reinheit 50%) in 10 ml wasserfreiem Dimethylformamid wurden 1,1 g 2-Acetamido-2-deoxy-3,4,6-tri-O-acetyl-α-D-glucopyranosylchlorid gegeben und weitere 3 Stunden gerührt. Danach wurden 30 ml Eiswasser und 50 ml Chloroform zugefügt und das Gemisch geschüttelt. Die Chloroformphase wurde abgetrennt, dreimal mit je 20 ml Eiswasser gewaschen, über Natriumsulfat getrocknet und unter vermindertem Druck eingeengt. Der Rückstand wurde in einer geringen Menge Ethylacetat gelöst und die Lösung auf eien Kieselgelsäule (30 g) gebracht. Die Säule wurde anschliessend mit Ethylacetat eluiert. Die Fraktionen 20—25 (jeweils 10 ml) wurden vereinigt und zu 406 mg einer festen Substanz eingeengt. Kristallisation dieser Substanz aus Ethylacetat-Hexan lieferte 301 mg (30% Ausbeute) 2′-(2-Acetamido-2-deoxy-3,4,6-tri-O-acetyl-β-D-glucopyranosyloxy)-4,4′,6′-trimethoxychalkon in Form hellgelber Kristalle; Fp. 108—110°C.

Beispiel 11

300 mg des gemäss Beispiel 10 erhaltenen 2′-(2-Acetamido-2-deoxy-3,4,6-tri-O-acetyl-β-D-glucopyranosyloxy)-4,4′,6′-trimethoxychalkons wurden in 4 ml Methanol gelöst. Der Lösung wurden 3 ml Triethylamin und 0,4 ml Wasser zugesetzt. Nach 8-stündigem Rühren bei Raumtemperatur wurde das Gemisch unter vermindertem Druck eingeengt. Kristallisation des Rückstandes aus Ethanol-Hexan lieferte 180 mg 2′-(2-Acetamido-2-deoxy-β-D-glucopyranosyloxy)-4,4′,6′-trimethoxychalkon in Form hellgelber Kristalle; Fp. 197—200°C.

Beispiel 12

Zu einer Lösung von 100 mg 2′-Hydroxy-4,4′,6′-Trimethoxychalkon in 2 ml Ethylacetat wurden 0,2 ml Triethylamin und 0,12 ml Bromacetylbromid zugesetzt. Es wurde 30 Minuten bei Raumtemperatur gerührt, mit 5 ml Dimethylformamid versetzt und weitere 3 Stunden gerührt. Danach wurden 50 ml Ether zugesetzt und das erhaltene Präzipitat wurde nach Abdekantieren des Ueberstandes in 5 ml Wasser gelöst.

Die Lösung wurde durch eine Dowex 1-Säule (Cl-Form, 1 x 20 cm) gegeben und lyophilisiert. Man erhielt 50 mg eines hellgelben hygroskopischen Puders, der nach Kristallisation aus Methanol-Ethylacetat 32 mg 2′,4,4′-Trimethoxy-6′-(triethylammonioacetoxy)-chalkon-chlorid lieferte: $^1H$—NMR ($D_2O$) δ1,4 (9H, $CH_2CH_3$), 3,8 (3H, $OCH_3$), 3,85 (6H, $OCH_3$) und 4,9 ppm (2H, $COCH_2N$).

Beispiel 13

Zu einer Lösung von 2′-Hydroxy-4,4′,6′-trimethoxychalkon in 5 ml wasserfreiem Tetrahydrofuran werden 10 ml Triethylamin, 43 mg p-(Dimethylamino)-pyridin und 182 mg 4-Methoxybenzoesäureanhydrid gegeben.

Nachdem 18 Stunden bei 80°C gerührt wurde, wurde das Gemisch eingeengt. 30 ml Ethylacetat wurden dem Rückstand zugesetzt und das Gemisch wurde nacheinander mit 0,2N Salzsäure, 10%igem Natriumcarbonat und Wasser gewaschen. Die Ethylacetatphase wurde abgetrennt, über Natriumsulfat getrocknet und eingeengt. Der Rückstand wurde dann an Kieselgel (6 g) chromatographiert, wobei mit Hexan-Ethylacetat (3:1 bis 1:1 v/v) eluiert wurde. Es wurden 180 mg eines hellgelben Oeles erhalten, das nach Kristallisation aus Methanol 110 mg 2′,4,4′-Trimethoxy-6′-(4-methoxybenzoyloxy)-chalkon in Form hellgelber Prismen lieferte; Fp. 111—111,5°C.

Beispiel 14

Zu einer Lösung von 156 mg Benzoylchlorid, 220 mg Triethylamin und 20 mg 4-(Dimethylamino)-pyridin in 5 ml wasserfreiem Tetrahydrofuran wurden 315 mg 2′-Hydroxy-4,4′,6′-trimethoxychalkon gegeben. Das Gemisch wurde kräftig bei Zimmertemperatur während 10 Minuten gerührt und dann in 50 ml eisgekühlte 0,1N Salzsäure gegossen.

Das Gemisch wurde zweimal mit je 70 ml Ethylacetat extrahiert, die Extrakte über Natriumsulfat getrocknet, eingeengt und der Rückstand aus Methanol umkristallisiert. Man erhielt 315 mg (75% Ausbeute) 2′-(Benzoyloxy)-4,4′,6′-trimethoxychalkon in Form hellgelber Nadeln; Fp. 133,8°C.

Beispiel 15

In zu Beispiel 14 analoger Weise wurde unter Verwendung von Benzyloxycarbonylchlorid 2′-(Benzyloxycarbonyloxy)-4,4′,6′-trimethoxychalkon in Form hellgelber Kristalle erhalten; Fp. 127,2°C.

Beispiel 16

In zu Beispiel 14 analoger Weise wurde unter Verwendung von 2,4-Dimethoxybenzoylchlorid 2′-(2,4-Dimethoxybenzoyloxy)-4,4′,6′-trimethoxychalkon in Form farbloser Prismen erhalten; Fp. 119—120°C.

Beispiel 17

In zu Beispiel 14 analoger Weise wurde unter Verwendung von 2-Furoylchlorid 2′-(2-Furoyloxy)-4,4′,6′-trimethoxychalkon in Form hellgelber Kristalle erhalten; Fp. 127,0°C.

Beispiel 18

Es wurde aus 5-Benzimidazolcarbaldehyd das 3-(5-Benzimidazolyl)-2′-hydroxy-4′,6′-dimethoxyacrylophenon in kristalliner Form erhalten; Fp. 147—149°C.

Beispiel 19

Ein Gemisch aus 207 ml 2′,4-Dihydroxy-4′,6′-dimethoxychalkon, 60 mg Natriumacetat und 70 mg Essigsäureanhydrid wurde 1 Stunde auf 90°C erhitzt. Das Gemisch wurde unter vermindertem Druck eingeengt und der Rückstand mit 30 ml Chloroform extrahiert.

Entfernung des Lösungsmittels und Umkristallisation des Rückstandes aus Methanol lieferte 157 mg 4-Acetoxy-2′-hydroxy-4′,6′-dimethoxychalkon; Fp. 141,8°C.

Beispiel 20

Tabletten enthaltend

| | |
|---|---|
| aktiven Wirkstoff (d.h. eine Verbindung der Formel I) | 300 mg |
| getrocknete Lactose | 200 mg |
| Cellulose, mikrokristallin | 30 mg |
| Polyvinylpyrrolidon | 5 mg |
| Magnesiumstearat | 4 mg |

können in an sich bekannter Weise hergestellt werden.

Beispiel 21

Tropfen für intranasale Applikation enthaltend pro 1 ml

| | |
|---|---|
| aktiven Wirkstoff (d.h. eine Verbindung der Formel I) | 0,1 mg |
| oberflächenaktives Mittel | 0,05 mg |
| Propylenglycol/Wasser (1:1, v/v) q.s. ad | 1 ml |

können unter Verwendung an sich bekannter Methoden hergestellt werden.

In derartigen Lösungen kann der aktive Wirkstoff in einer Konzentration von 0,001—1 mg/ml vorliegen.

Beispiel 22

Pastillen enthaltend

| | |
|---|---|
| aktiven Wirkstoff (d.h. eine Verbindung der Formel I) | 0,1 g |
| Sucrose, pulverisiert | 1,6 g |
| Acazia | 0,2 g |
| Dextrin | 0,1 g |
| Aromastoff | 0,001 g |

können in an sich bekannter Weise hergestellt werden.

**Patentansprüche für die Vertragsstaaten: BE CH DE FR GB IT LI LU NL SE**

1. Substituierte Acetophenone der allgemeinen Formel

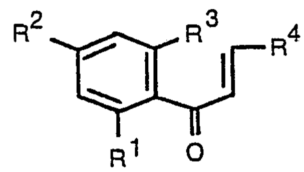

I

worin

$R^1$ Hydroxy, Phosphonooxy, Benzyloxycarbonyloxy, Trialkylammonioacyloxy, Furoyloxy, ggf.. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-, Niederalkoxy-, Alkylthio-, Alkylendioxy-, Acyloxy- oder Alkoxyalkoxy-substituiertes Phenyl darstellen, mit der Einschränkung, dass, wenn $R^1$ Hydroxy ist, $R^4$ Benzimidazolyl oder Acyloxy- substituiertes Phenyl bedeutet.

2. Eine Verbindung gemäss Anspruch 1 aus der Gruppe 2',4,4' - Trimethoxy - 6' - (phosphonooxy) - chalkon, 4 - Ethoxy - 2',4' - dimethoxy - 6' - (phosphonooxy) - chalkon, 2',4' - Dimethoxy-4 - methyl - 6' - (phosphonooxy) - chalkon, 2',4' - Dimethoxy - 4 - (methylthio) - 6' - (phosphonooxy)-chalkon, 2' - Ethoxy - 4,4' - dimethoxy - 6' - (phosphonooxy) - chalkon, 2',4' - Dimethoxy - 3 - (5-methyl - 2 - furyl) - 6' - (phosphonooxy) - acrylophenon, 4' - Ethoxy - 2',4 - dimethoxy - 6'-(phosphonooxy) - chalkon, 2',4' - Dimethoxy - 3,4 - (methylendioxy) - 6' - (phosphonooxy) - chalkon, 2',4' - Dimethoxy - 6' - (phosphonooxy) - 3 - (2 - thienyl) - acrylophenon, 2' - (2 - Acetamido - 2-deoxy-3,4,6 - tri - O - acetyl - $\beta$ - D - glucopyranosyloxy) - 4,4',6' - trimethoxychalkon, 2' - (2 - Acetamido - 2 - deoxy - $\beta$ - D - glucopyranosyloxy) - 4,4',6' - trimethoxychalkon, 2' - (Benzyloxycarbonyloxy)-4,4',6' - trimethoxychalkon, 2',4,4' - Trimethoxy - 6' - (triethylammonioacetoxy) - chalkon, 2',4,4'-Trimethoxy - 6' - (4 - methoxybenzoyloxy) - chalkon, 2',(2,4 - Dimethoxybenzoyloxy) - 4,4',6' - trimethoxychalkon, 2' - (Benzyloxy) - 4,4',6' - trimethoxychalkon, 2' - (2 - Furoyloxy) - 4,4',6' - trimethoxychalkon, 3 - (5 - Benzimidazolyl) - 2' - hydroxy - 4',6' - dimethoxyacrylophenon und 4-Acetoxy - 2' - hydroxy - 4',6' - dimethoxychalkon.

3. 4'-Ethoxy-2',4-dimethoxy-6'-(phosphonooxy)-chalkon.

4. Verfahren zur Herstellung substituierter Acetophenonderivate der allgemeinen Formel

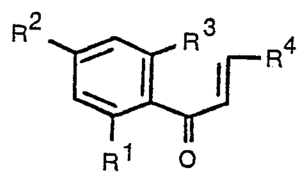

I

worin

$R^1$ Hydroxy, Phosphonooxy, Benzyloxycarbonyloxy, Trialkylammonioacyloxy, Furoyloxy, ggf. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-, Niederalkoxy-, Alkylthio-, Alkylendioxy-, Acyloxy- oder Alkoxyalkoxy-substituiertes Phenyl darstellen, mit der Einschränkung, dass, wenn $R^1$ Hydroxy ist, $R^4$ Benzimidazolyl oder Acyloxy- substituiertes Phenyl bedeutet,

dadurch gekennzeichnet, dass man

(a) eine Verbindung der Formel

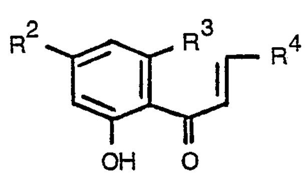

II

worin $R^2$, $R^3$ und $R^4$ die oben angegebenen Bedeutungen besitzen, mit einem Phosphoroxyhalogenid in einem Lösungsmittel in Gegenwart einer Base umsetzt und das Reaktionsprodukt hydrolysiert oder

(b) eine Verbindung der Formel II mit einem 2-Acetamido-2-deoxy-3,4,6-tri-O-acetyl-$\alpha$-D-gluco-

pyranosyl-halogenid in einem Lösungsmittel in Gegenwart eines Alkalimetallhalogenids umsetzt und gewünschtenfalls das Reaktionsprodukt partiell hydrolysiert oder

(c) eine Verbindung der Formel II mit einem Trialkylamin und Haloacetylhalogenid in einem Lösungsmittel umsetzt oder

(d) die Hydroxygruppe einer Verbindung der Formel II mit einem reaktiven Derivat einer Benzyloxycarbonsäure, einer ggf. substituierten Arylcarbonsäure oder einer Furancarbonsäure acyliert oder

(e) die Hydroxygruppe in 4-Stellung einer Verbindung der Formel

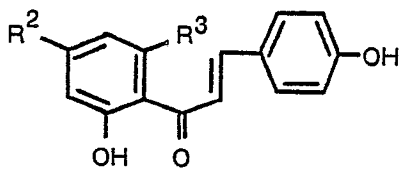

III

worin $R^2$ und $R^3$ die oben angegebenen Bedeutungen besitzen, mit einem reaktiven Derivat einer Alkancarbonsäure acyliert oder

(f) eine Verbindung der Formel

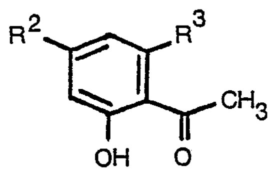

IV

worin $R^2$ und $R^3$ die oben angegebenen Bedeutungen besitzen, mit einem Aldehyd der Formel

$R^4$—CHO V

worin $R^4$ die oben angegebene Bedeutung besitzt, in einem Lösungsmittel in Gegenwart eines basischen Katalysators umsetzt.

5. Verfahren gemäss Anspruch 4, dadurch gekennzeichnet, dass eine Verbindung gemäss Anspruch 2 oder 3 hergestellt wird.

6. Substituierte Acetophenone der allgemeinen Formel

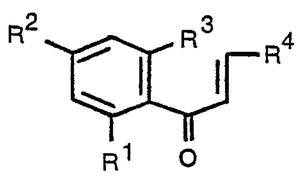

I

worin

$R^1$ Hydroxy, Phosphonooxy, Benzyloxycarbonyloxy, Trialkylammonioacyloxy, Furoyloxy, ggf. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-, Niederalkoxy-, Alkylthio-, Alkylendioxy-, Acyloxy- oder Alkoxyalkoxy-substituiertes Phenyl darstellen, mit der Einschränkung, dass, wenn $R^1$ Hydroxy ist, $R^4$ Benzimidazolyl oder Acyloxy- substituiertes Phenyl bedeutet, zwecks Verwendung als antivirale Mittel.

7. 4′-Ethoxy-2′,4-dimethoxy-6′-(phosphonooxy)-chalkon zwecks Verwendung als antivirales Mittel.

8. Pharmazeutische Präparate auf der Basis einer Verbindung der allgemeinen Formel

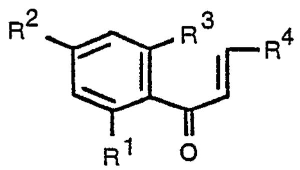

I

worin

$R^1$ Hydroxy, Phosphonooxy, Benzyloxycarbonyloxy, Trialkylammonioacyloxy, Furoyloxy, ggf. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-,

Niederalkoxy-, Alkylthio-, Alkylendioxy-, Acyloxy- oder Alkoxyalkoxy-substituiertes Phenyl darstellen, mit der Einschränkung, dass, wenn $R^1$ Hydroxy ist, $R^4$ Benzimidazolyl oder Acyloxy- substituiertes Phenyl bedeutet.

9. Pharmazeutische Präparate auf der Basis einer Verbindung gemäss Anspruch 2.

10. Pharmazeutische Präparate auf der Basis von 4'-Ethoxy-2',4-dimethoxy-6'-(phosphonooxy)-chalkon.

**Patentansprüche für den Vertragsstaat: AT**

1. Verfahren zur Herstellung substituierter Acetophenonderivate der allgemeinen Formel

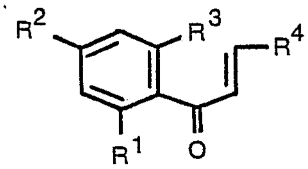

I

worin

$R^1$ Hydroxy, Phosphonooxy, Benzyloxycarbonyloxy, Trialkylammonioacyloxy, Furoyloxy, ggf. acyliertes Aminoglycosyloxy oder ggf. substituiertes Arylcarbonyloxy;

$R^2$ und $R^3$ Niederalkoxy und

$R^4$ Benzimidazolyl, ggf. Niederalkyl-substituiertes Furyl oder Thienyl oder ggf. Niederalkyl-, Niederalkoxy-, Alkylthio-, Alkylendioxy-, Acyloxy- oder Alkoxyalkoxy-substituiertes Phenyl darstellen, mit der Einschränkung, dass, wenn $R^1$ Hydroxy ist, $R^4$ Benzimidazolyl oder Acyloxy- substituiertes Phenyl bedeutet,

dadurch gekennzeichnet, dass man

(a) eine Verbindung der Formel

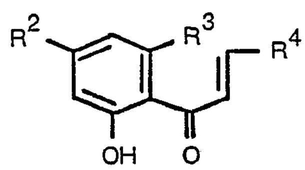

II

worin $R^2$, $R^3$ und $R^4$ die oben angegebenen Bedeutungen besitzen, mit einem Phosphoroxyhalogenid in einem Lösungsmittel in Gegenwart einer Base umsetzt und das Reaktionsprodukt hydrolysiert oder

(b) eine Verbindung der Formel II mit einem 2-Acetamido-2-deoxy-3,4,6-tri-O-acetyl-$\alpha$-D-glucopyranosyl-halogenid in einem Lösungsmittel in Gegenwart eines Alkalimetallhalogenids umsetzt und gewünschtenfalls das Reaktionsprodukt partiell hydrolysiert oder

(c) eine Verbindung der Formel II mit einem Trialkylamin und Haloacetylhalogenid in einem Lösungsmittel umsetzt oder

(d) die Hydroxygruppe einer Verbindung der Formel II mit einem reaktiven Derivat einer Benzyloxycarbonsäure, einer ggf. substituierten Arylcarbonsäure oder einer Furancarbonsäure acyliert oder

(e) die Hydroxygruppe in 4-Stellung einer Verbindung der Formel

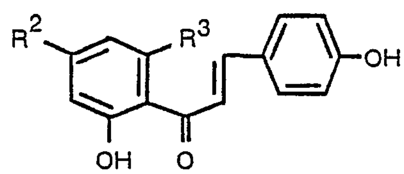

III

worin $R^2$ und $R^3$ die oben angegebenen Bedeutungen besitzen, mit einem reaktiven Derivat einer Alkancarbonsäure acyliert oder

(f) eine Verbindung der Formel

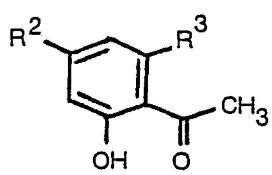

IV

worin $R^2$ und $R^3$ die oben angegebenen Bedeutungen besitzen, mit einem Aldehyd der Formel

$$R^4—CHO \quad V$$

worin $R^4$ die oben angegebene Bedeutung besitzt, in einem Lösungsmittel in Gegenwart eines basischen Katalysators umsetzt.

2. Verfahren gemäss Anspruch 1, dadurch gekennzeichnet, dass man 4′-Ethoxy-2′,4-dimethoxy-6′-(phosphonooxy)-chalkon herstellt.

**Claims for the Contracting States: BE CH DE FR GB IT LI LU NL SE**

1. Substituted acetophenones of the general formula

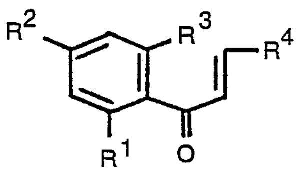

I

wherein

$R^1$ represents hydroxy, phosphonooxy, benzyloxycarbonyloxy, trialkylammonioacyloxy, furoyloxy, aminoglycosyloxy which may be acylated or arylcarbonyloxy which may be substituted;

$R^2$ and $R^3$ represent lower alkoxy and

$R^4$ represents benzimidazolyl, furyl or thienyl which may be lower alkyl-substituted or phenyl which may be lower alkyl-, lower alkoxy-, alkylthio-, alkylenedioxy-, acyloxy- or alkoxyalkoxy-substituted,

with the proviso that $R^4$ signifies benzimidazolyl or acyloxy-substituted phenyl when $R^1$ is hydroxy.

2. A compound according to claim 1 from the group 2′,4,4′ - trimethoxy - 6′ - (phosphonooxy)-chalcone, 4 - ethoxy - 2′,4′ - dimethoxy - 6′ - (phosphonooxy) - chalcone, 2′,4′ - dimethoxy - 4-methyl - 6′ - (phosphonooxy) - chalcone, 2′,4′ - dimethoxy - 4 - (methylthio) - 6′ - (phosphonooxy)-chalcone, 2′ - ethoxy - 4,4′ - dimethoxy - 6′ - (phosphonooxy) - chalcone, 2′,4′ - dimethoxy - 3 - (5-methyl - 2 - furyl) - 6′ - (phosphonooxy) - acrylophenone, 4′ - ethoxy - 2′,4 - dimethoxy - 6′ - (phosphonooxy) - chalcone, 2′,4′ - dimethoxy - 3,4 - (methylenedioxy) - 6′ - (phosphonooxy) - chalcone, 2′,4′ - dimethoxy - 6′ - (phosphonooxy) - 3 - (2 - thienyl) - acrylophenone, 2′ - (2 - acetamido - 2-deoxy - 3,4,6 - tri - O - acetyl - $\beta$ - D - glucopyranosyloxy - 4,4′,6′ - trimethoxychalcone, 2′ - (2 - acetamido - 2 - deoxy - $\beta$ - D - glucopyranosyloxy) - 4,4′,6′ - trimethoxychalcone, 2′ - (benzyloxycarbonyloxy) - 4,4′,6′ - trimethoxychalcone, 2′,4,4′ - trimethoxy - 6′ - (triethylammonioacetoxy)-chalcone, 2′,4,4′ - trimethoxy - 6′ - (4 - methoxybenzoyloxy) - chalcone, 2′,(2,4 - dimethoxybenzoyloxy)-4,4′,6′ - trimethoxychalcone, 2′ - (benzoyloxy) - 4,4′,6′ - trimethoxychalcone, 2′ - (2 - furoyloxy)-4,4′,6′ - trimethoxychalcone, 3 - (5 - benzimidazolyl) - 2′ - hydroxy - 4′,6′ - dimethoxyacrylophenone and 4 - acetoxy - 2′ - hydroxy - 4′,6′ - dimethoxychalcone.

3. 4′ -Ethoxy-2′,4 - dimethoxy - 6′ - (phosphonooxy) - chalcone.

4. Process for the manufacture of substituted acetophenone derivatives of the general formula

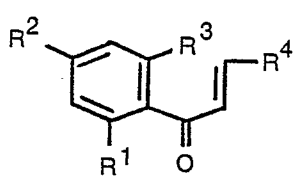

I

wherein $R^1$ represents hydroxy, phosphonooxy, benzyloxycarbonyloxy, trialkylammonioacyloxy, furoyloxy, aminoglycosyloxy which may be acylated or arylcarbonyloxy which may be substituted;

$R^2$ and $R^3$ represent lower alkoxy and

$R^4$ represents benzimidazolyl, furyl or thienyl which may be lower alkyl-substituted or phenyl which may be lower alkyl-, lower alkoxy-, alkylthio-, alkylenedioxy-, acyloxy- or alkoxyalkoxy-substituted,

with the proviso that $R^4$ signifies benzimidazolyl or acyloxy-substituted phenyl when $R^1$ is hydroxy, characterized by

(a) reacting a compound of the formula

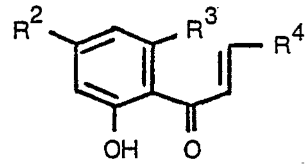

II

wherein $R^2$, $R^3$ and $R^4$ have the significances given above, with a phosphorus oxyhalide in a solvent in the presence of a base and hydrolyzing the reaction product, or

(b) reacting a compound of formula II with a 2-acetamido-2-deoxy-3,4,6-tri-O-acetyl-$\alpha$-D-glucopyranosyl halide in a solvent in the presence of an alkali metal halide and, if desired, partially hydrolyzing the reaction product, or

(c) reacting a compound of formula II with a trialkylamine and haloacetyl halide in a solvent, or

(d) acylating the hydroxy group in a compound of formula II with a reactive derivative of a benzyloxycarboxylic acid, an arylcarboxylic acid which may be substituted or a furan-carboxylic acid, or

(e) acylating the hydroxy group in the 4-position of a compound of the formula

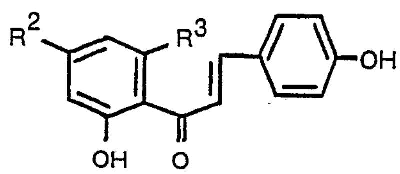

III

wherein $R^2$ and $R^3$ have the significances given above, with a reactive derivative of an alkanecarboxylic acid, or

(f) reacting a compound of the formula

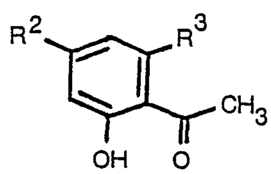

IV

wherein $R^2$ and $R^3$ have the significances given above, with an aldehyde of the formula

$$R^4\text{—CHO} \qquad V$$

wherein $R^4$ has the significance given above, in a solvent in the presence of a basic catalyst.

5. Process according to claim 4, characterized in that a compound according to claim 2 or 3 is manufactured.

6. Substituted acetophenones of the general formula

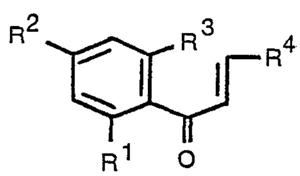

I

wherein

$R^1$ represents hydroxy, phosphonooxy, benzyloxycarbonyloxy, trialkylammonioacyloxy, furoyloxy, aminoglycosyloxy which may be acylated or arylcarbonyloxy which may be substituted;

$R^2$ and $R^3$ represent lower alkoxy and

$R^4$ represents benzimidazolyl, furyl or thienyl which may be lower alkyl-substituted or phenyl which may be lower alkyl-, lower alkoxy-, alkylthio-, alkylenedioxy-, acyloxy- or alkoxyalkoxy-substituted, with the proviso that $R^4$ signifies benzimidazolyl or acyloxy-substituted phenyl when $R^1$ is hydroxy, for use as antiviral agents.

7. 4′-Ethoxy-2′,4-dimethoxy-6′-(phosphonooxy)-chalcone for use as an antiviral agent.

8. Pharmaceutical preparations based on a compound of the general formula

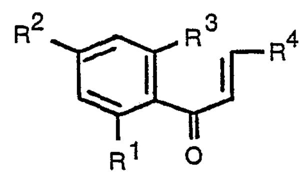

I

wherein

$R^1$ represents hydroxy, phosphonooxy, benzyloxycarbonyloxy, trialkylammonioacyloxy, furoyloxy, aminoglycosyloxy which may be acylated or arylcarbonyloxy which may be substituted;

$R^2$ and $R^3$ represent lower alkoxy and

$R^4$ represents benzimidazolyl, furyl or thienyl which may be lower alkyl-substituted or phenyl which may be lower alkyl-, lower alkoxy-, alkylthio-, alkylenedioxy-, acyloxy- or alkoxyalkoxy-substituted, with the proviso that $R^4$ signifies benzimidazolyl or acyloxy-substituted phenyl when $R^1$ is hydroxy.

9. Pharmaceutical preparations based on a compound according to claim 2.

10. Pharmaceutical preparations based on 4′-ethoxy-2′,4-dimethoxy-6′-(phosphonooxy)-. chalcone.

**Claims for the Contracting State: AT**

1. Process for the manufacture of substituted acetophenone derivatives of the general formula

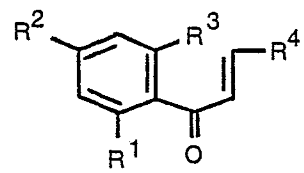

I

wherein

$R^1$ represents hydroxy, phosphonooxy, benzyloxycarbonyloxy, trialkylammonioacyloxy, furoyloxy, aminoglycosyloxy which may be acylated or arylcarbonyloxy which may be substituted;

$R^2$ and $R^3$ represent lower alkoxy and

$R^4$ represents benzimidazolyl, furyl or thienyl which may be lower alkyl-substituted or phenyl which may be lower alkyl-, lower alkoxy-, alkylthio-, alkylenedioxy-, acyloxy or alkoxyalkoxy-substituted, with the proviso that $R^4$ signifies benzimidazolyl or acyloxy-substituted phenyl when $R^1$ is hydroxy, characterized by

(a) reacting a compound of the formula

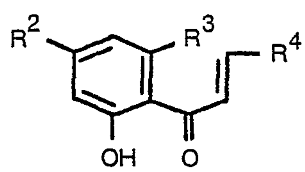

II

wherein $R^2$, $R^3$ and $R^4$ have the significance given above,
with a phosphorus oxyhalide in a solvent in the presence of a base and hydrolyzing the reaction product, or

(b) reacting a compound of formula II with a 2-acetamido-2-deoxy-3,4,6-tri-O-acetyl-$\alpha$-D-glucopyranosyl halide in a solvent in the presence of an alkali metal halide and, if desired, partially hydrolyzing the reaction product, or

(c) reacting a compound of formula II with a trialkylamine and haloacetyl halide in a solvent, or

(d) acylating the hydroxy group in a compound of formula II with a reactive derivative of a benzyloxycarboxylic acid, an arylcarboxylic acid which may be substituted or a furan-carboxylic acid, or

(e) acylating the hydroxy group in the 4-position of a compound of the formula

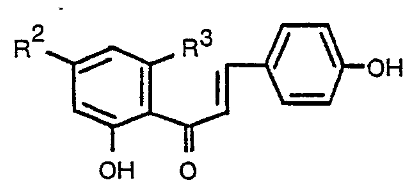

III

wherein $R^2$ and $R^3$ have the significances given above, with a reactive derivative of an alkanecarboxylic acid, or

(f) reacting a compound of the formula

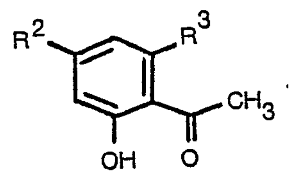

IV

wherein $R^2$ and $R^3$ have the significances given above, with an aldehyde of the formula

$R^4$—CHO V

wherein $R^4$ has the significance given above, in a solvent in the presence of a basic catalyst.

2. Process according to claim 1, characterized in that 4′-ethoxy-2′,4-dimethoxy-6′-(phosphonooxy)-chalcone is manufactured.

**Revendications pour les Etats contractants: BE CH DE FR GB IT LI LU NL SE**

1. Acétophénones substituées de formule générale

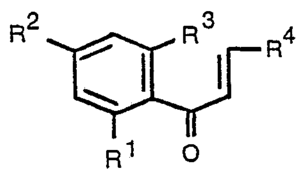

I

où

$R^1$ représente un hydroxy, un phosphonooxy, un benzyloxycarbonyloxy, un trialcoylammonioacyloxy, un furoyloxy, un aminoglycosyloxy éventuellement acylé ou un arylcarbonyloxy éventuellement substitué;

$R^2$ et $R^3$ représentent un alcoxy inférieur et

$R^4$ représente un benzimidazolyle, un furyle ou un thiényle éventuellement substitué par un alcoyle inférieur ou un phényle éventuellement substitué par un alcoyle inférieur, un alcoxy inférieur, un alcoylthio, un alcoylènedioxy, un acyloxy ou un alcoxyalcoxy,

avec cette limitation que lorsque $R^1$ est un hydroxy,

$R^4$ représente un benzimidazolyle ou un phényle substitué par un acyloxy.

2. Composé selon la revendication 1 du groupe 2′,4,4′ - triméthoxy - 6′ - (phosphonooxy)-chalcone, 4 - éthoxy - 2′,4′ - diméthoxy - 6′ - (phosphonooxy) - chalcone, 2′,4′ - diméthoxy - 4-méthyl - 6′ - (phosphonooxy) - chalcone, 2′,4′ - diméthoxy - 4 - (méthylthio) - 6′ - (phosphonooxy)-chalcone, 2′ - éthoxy - 4,4′ - diméthoxy - 6′ - (phosphonooxy) - chalcone, 2′,4′ - diméthoxy - 3-(5 - méthyl - 2 - furyl) - 6′ - (phosphonooxy) - acrylophénone, 4′ - éthoxy - 2′,4′ - diméthoxy - 6′-(phosphonooxy) - chalcone, 2′,4′ - diméthoxy - 3,4 - (méthylènedioxy) - 6′ - (phosphonooxy) - chalcone, 2′,4′ - diméthoxy - 6′ - (phosphonooxy) - 3 - (2 - thiényl)-acrylophénone, 2′ - (2 - acétamido-2 - désoxy - 3,4,6 - tri - O - acétyl - $\beta$ - D - glucopyranosyloxy) - 4,4′,6′ - triméthoxychalcone, 2′-(2 - acétamido - 2 - désoxy - $\beta$ - D - glucopyranosyloxy) - 4,4′,6′ - triméthoxy - chalcone, 2′ - (benzyloxycarbonyloxy) - 4,4′,6′ - triméthoxy - chalcone, 2′,4,4′ - triméthoxy - 6′ - (triéthylammonioacétoxy)-chalcone, 2′,4,4′ - triméthoxy - 6′ - (4 - méthoxybenzoyloxy) - chalcone, 2′ - (2,4 - diméthoxy-benzoyloxy) - 4,4′,6′ - triméthoxychalcone, 2′ - (benzoyloxy) - 4,4′,6′ - triméthoxychalcone, 2′-(2 - furoyloxy) - 4,4′,6′ - triméthoxychalcone, 3 - (5 - benzimidazolyl) - 2′ - hydroxy - 4′,6′ - diméthoxy-acrylophénone et 4 - acétoxy - 2′ - hydroxy - 4′,6′ - diméthoxychalcone.

3. 4′-éthoxy-2′,4-diméthoxy-6′-(phosphonooxy)-chalcone.

4. Procédé de préparation de dérivés d'acétophénone substitués de formule générale

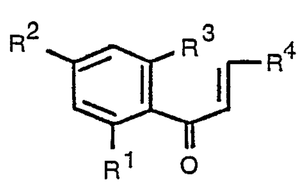

I

où

$R^1$ représente un hydroxy, un phosphonooxy, un benzyloxycarbonyloxy, un

trialcoylammonioacyloxy, un furoyloxy, un aminoglycosyloxy, éventuellement acylé ou un arylcarbonyloxy éventuellement substitué;

$R^2$ et $R^3$ représentent un alcoxy inférieur et

$R^4$ représente un benzimidazolyle, un furyle ou un thiényle éventuellement substitué par un alcoyle inférieur ou un phényle éventuellement substitué par un alcoyle inférieur, un alcoxy inférieur, un alcoylthio, un alcoylènedioxy, un acyloxy ou un alcoxyalcoxy, avec cette limitation que lorsque $R^1$ est un hydroxy, $R^4$ représente un benzimidazolyle ou un phényle substitué par un acyloxy,
caractérisé en ce que

(a) on fait réagir un composé de formule

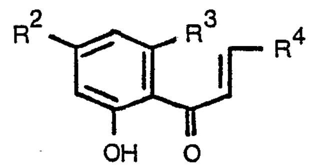

II

où $R^2$, $R^3$ et $R^4$ ont les significations données ci-dessus, avec un oxyhalogénure de phosphore dans un solvant en présence d'une base et on hydrolyse le produit de la réaction ou

(b) on fait réagir un composé de formule II avec un halogénure de 2-acétamido-2-désoxy-3,4,6-tri-O-acétyl-$\alpha$-D-glucopyranosyle dans un solvant en présence d'un halogénure de métal alcalin et si on le désire on hydrolyse partiellement le produit de la réaction ou

(c) on fait réagir un composé de formule II avec une trialcoylamine et un halogénure d'haloacétyle dans un solvant ou

(d) on acyle le groupe hydroxy d'un composé de formule II avec un dérivé réactif d'un acide benzyloxycarboxylique, d'un acide arylcarboxylique éventuellement substitué ou d'une acide furannecarboxylique ou

(e) on acyle le groupe hydroxy en position 4 d'un composé de formule

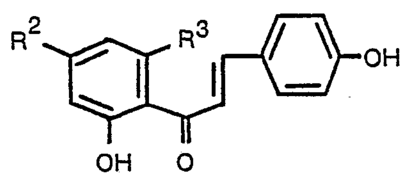

III

où $R^2$ et $R^3$ ont les significations données ci-dessus, avec un dérivé réactif d'un acide alcanecarboxylique ou

(f) on fait réagir un composé de formule

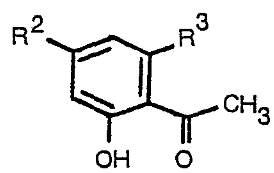

IV

où $R^2$ et $R^3$ ont les significations données ci-dessus, avec un aldéhyde de formule

$$R^4\text{—CHO} \qquad V$$

où $R^4$ a la signification donnée ci-dessus, dans un solvant en présence d'un catalyseur basique.

5. Procédé selon la revendication 4, caractérisé en ce qu'on prépare un composé selon l'une des revendications 2 ou 3.

6. Acétophénones substituées de formule générale

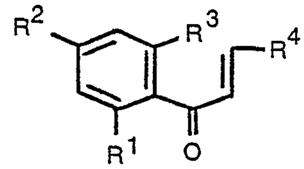

I

où $R^1$ représente un hydroxy, un phosphonooxy, un benzyloxycarbonyloxy, un trialcoylammonioacyloxy, un furoyloxy, un aminoglycosyloxy éventuellement acylé ou un arylcarbonyloxy éventuellement substitué;

$R^2$ et $R^3$ représentent un alcoxy inférieur et

$R^4$ représente un benzimidazolyle, un furyle ou un thiényle éventuellement substitué par un alcoyle inférieur ou un phényle éventuellement substitué par un alcoyle inférieur, un alcoxy inférieur, un alcoylthio, un alcoylènedioxy, un acyloxy ou un alcoxyalcoxy,

avec cette limitation que lorsque $R^1$ est un hydroxy, $R^4$ représente un benzimidazolyle ou un phényle substitué par un alcoxy,

aux fins d'application comme agents antiviraux.

7. 4'-éthoxy-2',4-diméthoxy-6'-(phosphonooxy)-chalcone aux fins d'application comme agent anti-viral.

8. Préparations pharmaceutique à base d'un composé de formule générale

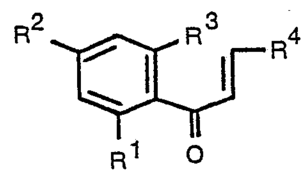

I

où $R^1$ représente un hydroxy, un phosphonooxy, un benzyloxycarbonyloxy, un trialcoylammonioacyloxy, un furoyloxy, un aminoglycosyloxy éventuellement acylé ou un arylcarbonyloxy éventuellement substitué;

$R^2$ et $R^3$ représentent un alcoxy inférieur et

$R^4$ représente un benzymidazolyle, un furyle ou un thiényle éventuellement substitué par un alcoyle inférieur ou un phényle éventuellement substitué par un alcoyle inférieur, un alcoxy inférieur, un alcoylthio, un alcoylènedioxy, un acyloxy ou un alcoxyalcoxy,

avec cette limitation que lorsque $R^1$ est un hydroxy, $R^4$ représente un benzimidazolyle ou un phényle substitué par un acyloxy.

9. Préparations pharmaceutiques à base d'un composé selon la revendication 2.

10. Préparations pharmaceutiques à base de 4'-éthoxy-2',4-diméthoxy-6'-(phosphonooxy)-chalcone.

**Revendications pour l'Etat contractant: AT**

1. Procédé de préparation de dérivés d'acétophénone substitués de formule générale

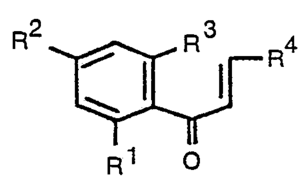

I

où

$R^1$ représente un hydroxy, un phosphonooxy, un benzyloxycarbonyloxy, un trialcoylammonioacyloxy, un furoyloxy, un aminoglycosyloxy éventuellement acylé ou un arylcarbonyloxy éventuellement substitué;

$R^2$ et $R^3$ représentent un alcoxy inférieur et

$R^4$ représente un benzimidazolyle, un furyle ou un thiényle éventuellement substitué par un alcoyle inférieur ou un phényle éventuellement substitué par un alcoyle inférieur, un alcoxy inférieur, un alcoylthio, un alcoylènedioxy, un acyloxy ou un alcoxyalcoxy,

avec cette limitation que lorsque $R^1$ est un hydroxy, $R^4$ représente un benzimidazolyle ou un phényle substitué par un acyloxy,

caractérisé en ce que

(a) on fait réagir un composé de formule

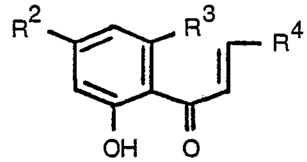

II

où $R^2$, $R^3$ et $R^4$ ont les significations données ci-dessus, avec un oxyhalogénure de phosphore dans un solvant en présence d'une base et on hydrolyse le produit de la réaction ou

(b) on fait réagir un composé de formule II avec un halogénure de 2-acétamido-2-désoxy-3,4,6-

tri-O-acétyl-$\alpha$-D-glucopyranosyle dans un solvant en présence d'un halogénure de métal alcalin et si on le désire on hydrolyse partiellement le produit de la réaction ou

(c) on fait réagir un composé de formule II avec une trialcoylamine et un halogénure d'haloacétyle dans un solvant ou

(d) on acyle le groupe hydroxy d'un composé de formule II avec un dérivé réactif d'un acide benzyloxycarboxylique, d'un acide arylcarboxylique éventuellement substitué ou d'un acide furannecarboxylique ou

(e) on acyle le groupe hydroxy en position 4 d'un composé de formule

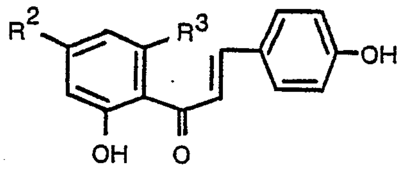

III

où $R^2$ et $R^3$ ont les significations données ci-dessus, avec un dérivé réactif d'un acide alcanecarboxylique ou

(f) on fait réagir un composé de formule

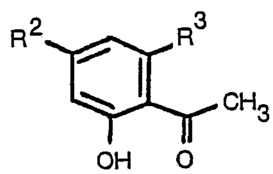

IV

où $R^2$ et $R^3$ ont les significations données ci-dessus, avec un aldéhyde de formule

$$R^4\text{—CHO} \qquad V$$

où $R^4$ a la signification donnée ci-dessus, dans un solvant en présence d'un catalyseur basique.

2. Procédé selon la revendication 1, caractérisé en ce qu'on prépare la 4'-éthoxy-2',4-diméthoxy-6'-(phosphonooxy)-chalcone.